# EUROPÄISCHE PATENTSCHRIFT

(11) **EP 1 523 356 B1**
(45) Veröffentlichungstag und Bekanntmachung des Hinweises auf die Patenterteilung: **20.08.2008**
(21) Anmeldenummer: 03763565.3
(22) Anmeldetag: 08.07.2003
(51) Int. Cl.: A61M 5/24

(54) **VERABREICHUNGSGERÄT MIT RÜCKZUGSGESPERRTER KOLBENSTANGE**
ADMINISTRATION DEVICE COMPRISING A PLUNGER ROD WITH A RETURN LOCK
DISPOSITIF D'ADMINISTRATION PRESENTANT UNE TIGE DE PISTON A RETOUR BLOQUE

(30) Priorität: 16.07.2002 DE 10232158
(43) Veröffentlichungstag der Anmeldung: 20.04.2005
(73) Patentinhaber: TecPharma Licensing AG, 3401 Burgdorf (CH)
(72) Erfinder: KIRCHHOFER, Fritz, CH-3454 Sumiswald (CH); GURTNER, Thomas, CH-3425 Koppigen (CH)
(86) Internationale Anmeldenummer: PCT/CH2003/000459
(87) Internationale Veröffentlichungsnummer: WO 2004/006997

(56) Entgegenhaltungen:
- EP-A- 0 327 910
- WO-A-96/07443
- WO-A-97/36623
- DE-C- 19 900 827
- US-A- 5 611 783

## Beschreibung

Die Erfindung betrifft ein Verabreichungsgerät für die Verabreichung eines fluiden Produkts. Das Haupteinsatzgebiet ist die Human-Medizin. Das erweiterte Einsatzgebiet umfasst jedoch auch die Kosmetik und die Tiermedizin. Insbesondere betrifft die Erfindung ein Verabreichungsgerät für die Selbstverabreichung, mit dem ein Verwender des Geräts das betreffende Produkt sich selbst verabreicht. Die Selbstverabreichung ist beispielsweise in der Diabetestherapie oder in der Verabreichung von Wachstumshormonen üblich, um nur zwei prominente Beispiele zu nennen.

Durchgesetzt haben sich insbesondere für die Selbstverabreichung Injektionsgeräte in Form von sogenannten Injektions-Pens, bei denen das Produkt mit Hilfe eines Kolbens aus einem Produktreservoir ausgeschüttet und verabreicht wird. Der Kolben wird über eine Kolbenstange betätigt. Die Kolbenstange ist mit einer Antriebsvorrichtung in einem Eingriff, um durch eine Betätigung der Antriebsvorrichtung die Kolbenstange und damit zusammen den Kolben in eine Vorschubrichtung zu bewegen und dadurch Produkt auszuschütten. Zahnstangen sind ein gebräuchlicher Typ von Kolbenstangen. Zahnstangen weisen wenigstens eine in Längsrichtung der Zahnstange erstreckte Zahnreihe auf. Die wenigstens eine Zahnreihe muss die Bewegung der Kolbenstange in die Vorschubrichtung zulassen und kann den Eingriff mit der Antriebsvorrichtung bilden. Eine Kolbenstange wird üblicherweise dann als Zahnstange ausgebildet, wenn eine Bewegung der Kolbenstange entgegen der Vorschubrichtung nicht zugelassen werden soll. Hierfür wirkt die Zahnreihe mit einer Sperreinrichtung zusammen, die in die Zahnreihe eingreift und dadurch eine Rückzugsbewegung der Kolbenstange in jeder axialen Position, welche die Kolbenstange im ungestörten Betrieb einnehmen kann, verhindert. Wegen der Funktion der wenigstens einen Zahnreihe als Rückzugssperre, sind Verabreichungsgeräte mit Kolbenstangen, die als Zahnstangen ausgebildet sind, typischerweise Geräte, die nach der vollständigen Entleerung des Reservoirs im Ganzen oder zu einem Teil entsorgt werden.

Um den korrekten Einbau einer als Zahnstange ausgebildeten Kolbenstange zu gewährleisten, sollte solch eine Kolbenstange nur von dem Gerätehersteller selbst in das Gerät eingebaut werden. Wegen des kritischen Zusammenspiels von Rückzugssperre einerseits und dem Eingriff der Antriebsvorrichtung oder gar einer Dosiermechanik in die Kolbenstange, können beim Einbau solch einer Kolbenstange Fehler auftreten, insbesondere wenn solch ein Einbau von einem Verwender selbst vorgenommen wird. Der Einbau einer Kolbenstange durch den Verwender selbst birgt die Gefahr, dass die Kolbenstange in Bezug auf die mit ihr in Eingriff zu bringenden Teile des Verabreichungsgerätes nicht korrekt eingebaut wird.

Es ist daher eine Aufgabe der Erfindung, noch sicherer als bei den bekannten Verabreichungsgeräten zu verhindern, dass ein Verwender Manipulationen an und mit der Kolbenstange seines Verabreichungsgeräts vornehmen kann.

Die Erfindung betrifft eine Vorrichtung zur Verabreichung ein injizierbaren Produkts, die ein Gehäuse mit einem Reservoir für das Produkt, einen in dem Reservoir in eine Vorschubrichtung verschiebbaren Kolben, eine in die Vorschubrichtung bewegbare, auf den Kolben wirkende Kolbenstange und eine Sperrmechanik umfasst, die dazu dient, eine gegen die Vorschubrichtung gerichtete Bewegung der Kolbenstange zu verhindern. Die Sperrmechanik umfasst oder besteht aus einer von der Kolbenstange gebildeten ersten Sperreinrichtung und einer relativ zu dem Gehäuse gegen die Vorschubrichtung nicht bewegbaren zweiten Sperreinrichtung, die permanent miteinander im Eingriff sind. Die zweite Sperreinrichtung ist relativ zu dem Gehäuse vorzugsweise auch nicht in die Vorschubrichtung bewegbar.

Die erste Sperreinrichtung ist vorzugsweise in einem Stück mit der Kolbenstange geformt. Die Kolbenstange kann insbesondere aus Kunststoff in einem Verfahren der Urformung, vorzugsweise als Spritzgussteil, gefertigt sein. Die zweite Sperreinrichtung kann separat von dem Gehäuse gebildet und mit dem Gehäuse geeignet verbunden sein, vorzugsweise ist sie jedoch in einem Stück unmittelbar mit einem Gehäuseabschnitt geformt. Auch sie kann insbesondere aus Kunststoff in einem hierfür geeigneten Verfahren der Urformung, insbesondere dem Spritzguss, gebildet sein.

Die eine der Sperreinrichtungen umfasst wenigstens eine Reihe von in Vorschubrichtung hintereinander angeordneten, ersten Sperrelementen. Die Reihe der ersten Sperrelemente wird im Folgenden als Sperrelementreihe bezeichnet. Die andere der Sperreinrichtungen umfasst wenigstens ein zweites Sperrelement, das in eine einzige Lücke oder mehrere Lücken eingreift, die zwischen benachbarten ersten Sperrelementen in der Sperrelementreihe verbleibt oder verbleiben. Die Sperrelementreihe kann insbesondere eine Zahnreihe, vorzugsweise eine Sägezahnreihe sein, wie dies von bekannten Zahnstangen bekannt ist. Die Sperrelementreihe und das zweite Sperrelement bilden eine Rückzugssperre, die auf dem Prinzip des Widerhakens beruht, um den Rückzug der Kolbenstange, d. h. die gegen die Vorschubrichtung gerichtete Bewegung, zu verhindern.

Um die Bewegung in die Vorschubrichtung zu ermöglichen, kann das wenigstens eine zweite Sperrelement gegen eine Elastizitätskraft aus dem Eingriff mit der Sperrelementreihe bewegt werden. Der Rückzugsbewegung der Kolbenstange wirkt der Eingriff zwischen dem zweiten Sperrelement und der Sperrelementreihe jedoch mit einer Widerstandskraft entgegen, die deutlich größer ist als die für die Vorschubbewegung zu überwindende Elastizitätskraft. Zur Erzeugung der Elastizitätskraft ist das zweite Sperrelement vorzugsweise biegeelastisch gebildet. Grundsätzlich wäre es jedoch auch möglich, das zweite Sperrelement an einem separaten Federelement elastisch abzustützen. Die Widerstandskraft zur Verhinderung der Rückzugsbewegung beruht auf einem Formschluss zwischen der Sperrelementreihe und dem zweiten Sperrelement.

Da durch das Zusammenwirken der Sperrelementreihe und des zweiten Sperrelements zwar die Rückzugsbewegung der Kolbenstange verhindert, aber die Bewegung in die Vorschubrichtung zugelassen werden soll, sind der die Rückzugsbewegung verhindernden Widerstandskraft jedoch Grenzen gesetzt. Es ist daher durchaus vorstellbar, dass ein Verwender die Kolbenstange trotz des Zusammenwirkens der beiden Sperreinrichtungen zurückzieht oder gar so weit zurückzieht, dass die Sperreinrichtungen gänzlich außer Eingriff gelangen.

Erfindungsgemäß wird dies dadurch verhindert, dass entweder die erste Sperreinrichtung an ihrem in Vorschubrichtung vorderen Ende oder die zweite Sperreinrichtung an ihrem in Vorschubrichtung hinteren Ende ein Sperrsicherungselement bildet, das einer gegen die Vorschubrichtung gerichteten Bewegung der Kolbenstange eine Sperrkraft entgegensetzt, die größer als die genannte Widerstandskraft ist. Es kann auch je ein solches Sperrsicherungselement an dem vorderen Ende der ersten Sperreinrichtung und an dem hinteren Ende der zweiten Sperreinrichtung gebildet sein, obgleich dies nicht erforderlich ist. Die erste und/oder die zweite Sperreinrichtung kann oder können auch je mehrere Sperrsicherungselemente umfassen.

Das Sperrsicherungselement ist vorzugsweise an einem Ende der Sperrelementreihe gebildet. Die Sperrelementreihe ist vorzugsweise an der Kolbenstange gebildet, d. h. die Sperrelementreihe bildet vorzugsweise die erste Sperreinrichtung oder zumindest einen Teil der ersten Sperreinrichtung. Falls das Sperrsicherungselement an der Kolbenstange gebildet ist, vorzugsweise in einem Stück mit der Kolbenstange geformt, so ist es ungeachtet der Frage, ob die erste Sperreinrichtung die Sperrelementreihe oder das zweite Sperrelement bildet, vor der so gebildeten Sperrelementreihe oder dem so gebildeten zweiten Sperrelement angeordnet. Ist das Sperrsicherungselement Bestandteil der zweiten Sperreinrichtung, so ist es ungeachtet der Frage, ob die zweite Sperreinrichtung die Sperrelementreihe oder das zweite Sperrelement bildet, hinter der so gebildeten Sperrelementreihe oder hinter dem so gebildeten zweiten Sperrelement.

Zur Erzeugung der Sperrkraft wirkt das Sperrsicherungselement mit einem Sicherungsgegenelement zusammen, um ein Lösen der Kolbenstange von dem Gehäuse oder Gehäuseabschnitt zu verhindern. In bevorzugter Ausführung wirkt es entweder mit der Sperrelementreihe oder noch bevorzugter mit dem zweiten Sperrelement zusammen, so dass nicht noch ein zusätzliches Sicherungsgegerielement lediglich für einen Eingriff zur Erzeugung der Sperrkraft benötigt wird, obgleich auch solch eine Ausbildung nicht ausgeschlossen werden soll. Das Sperrsicherungselement kann insbesondere in der Art der die Sperrelementreihe bildenden ersten Sperrelemente gebildet sein, wodurch ein Zusammenwirken mit dem zweiten Sperrelement erleichtert wird. Einer bevorzugten Ausführung entspricht es aber durchaus auch, wenn das Sperrsicherungselement in der Art des zweiten Sperrelements gebildet wird, um mit der Sperrelementreihe zusammenwirken zu können.

Um den Einbau der Kolbenstange zu erleichtern, wird es bevorzugt, wenn das Sperrsicherungselement die Bewegung der Kolbenstange in die Vorschubrichtung zulässt. Das Sperrsicherungselement sollte demnach mit dem Sicherungsgegenelement dem Grunde nach wie die Sperrelementreihe und das zweite Sperrelement zusammenwirken. Zwischen dem Sperrsicherungselement und dem zur Erzeugung der Sperrkraft zusammenwirkenden Sicherungsgegenelement sollte eine elastische Nachgiebigkeit vorhanden sein, die ein Abgleiten des Sicherungsgegenelements an dem Sicherungsgegenelement gestattet, wenn die Kolbenstange in die Vorschubrichtung bewegt wird. Die Kraft, die aufgebracht werden muss, damit das Sperrsicherungselement und das Sicherungsgegenelement bei der Vorschubbewegung der Kolbenstange aneinander abgleiten wird wohl größer sein als die genannte Elastizitätskraft, sollte andererseits jedoch deutlich kleiner sein als die genannte Widerstandskraft.

Das Sperrsicherungselement ist in Bezug auf die Sperrelementreihe und das zweite Sperrelement vorzugsweise so angeordnet, dass es verhindert, dass die Sperrelementreihe und das zweite Sperrelement aus ihrem Eingriff gebracht werden können. Falls das Sperrsicherungselement an einem der beiden Enden der Sperrelementreihe angeordnet ist, sollte es von dem nächst benachbarten der ersten Sperrelemente in Vorschubrichtung am besten den gleich Abstand aufweisen, wie je zwei hintereinander nächst benachbarte erste Sperrelemente um eine stets definierte Axialposition der Kolbenstange zu garantieren. Besonders bevorzugt ist das Sperrsicherungselement in der Verlängerung der Sperrelementreihe angeordnet, da in diesem Fall das zweite Sperrelement zur Erzeugung der Sperrkraft mit dem Sperrsicherungselement in gleicher Weise wie mit den ersten Sperrelementen zusammenwirken kann. Falls das Sperrsicherungselement in der Art des zweiten Sperrelements gebildet ist und die Sperrkraft im Zusammenwirken mit der Sperrelementreihe erzeugt, sollten das zweite Sperrelement und das Sperrsicherungselement in Vorschubrichtung zueinander einen Abstand aufweisen, der der Länge der Sperrelementreihe entspricht.

In einer Ausführungsform weist die Kolbenstange einen Gewindeabschnitt auf, um in einem Gewindeeingriff mit einer Gewindemutter eine Dosiermechanik oder einen Teil einer Dosiermechanik zu bilden. In dem Gewindeabschnitt ist in bevorzugter Ausführung eine sich in Vorschubrichtung erstreckende Ausnehmung gebildet, in der eine der Sperreinrichtungen, vorzugsweise die Sperrelementreihe, angeordnet ist. Vorzugsweise ist auch das Sperrsicherungselement in der Ausnehmung angeordnet. Die Sperreinrichtung und das Sperrsicherungselement, falls ein solches in der Ausnehmung angeordnet ist, ragen in radialer Richtung bis höchstens zu dem Gewindegrund, vorzugsweise stehen sie radial ein Stück weit hinter dem Gewindegrund zurück. Falls die Ausnehmung sich in oder gegen die Vorschubrichtung über den Gewindeabschnitt hinaus erstreckt und die betreffende Sperreinrichtung und/oder das Sperrsicherungselement in der Vertiefung außerhalb des Gewindeabschnitts angeordnet sind, gilt dies gleichermaßen, d. h. die betreffende Sperreinrichtung und/oder das Sperrsicherungselement stehen nicht über eine den Gewindegrund verlängernde Mantelaußenfläche der Kolbenstange vor. Grundsätzlich kann die Kolbenstange jedoch auch als eine Zahnstange ohne Gewinde oder auch mit anderen Mitteln für die Dosierung gebildet sein.

Die Erfindung kann bei jeder Art von Verabreichungsgeräten verwirklicht sein die auf einer Produktausschüttung mittels Kolben und Kolbenstange beruhen. Besonders vorteilhaft, findet sie jedoch Verwendung bei Geräten, bei denen ein aufgebrauchtes Produktreservoir nicht wieder nachgefüllt wird. Vorteilhaft ist die Erfindung insbesondere für sogenannte Semi-disposable Pens. Dies sind Injektionsgeräte, die ein Reservoirmodul und ein Dosier- und Betätigungsmodul aufweisen, wobei sie vorzugsweise bereits aus diesen beiden Modulen allein bestehen. Das Reservoirmodul wird nach dem Aufbrauch des in seinem Reservoir enthaltenen Produkts entsorgt, während das Dosier- und Betätigungsmodul für die Verwendung mit stets wieder neuen Reservoirmodulen vorgesehen ist. In dem Dosier- und Betätigungsmodul sind die hochwertigen und daher hochpreisigen Teile des Verarbeitungsgerätes zusammengefasst. Da das Reservoirmodul nicht für unbegrenzt viele Vorgänge der Dosisauswahl und Produktausschüttung gedacht ist, können die in ihm zusammengefassten Teile preiswert hergestellt werden. Die Aufspaltung des Verabreichungsgerätes in ein nur für eine begrenzte Anzahl von Produktausschüttungen zur Verfügung stehendes Reservoirmodul und ein in Bezug auf die Anzahl der Dosiervorgänge und Produktausschüttungen nicht begrenztes Dosier- und Betätigungsmodul eröffnet neue Möglichkeiten für die Optimierung des Kosten/Nutzen-Verhältnisses solcher Geräte.

Bevorzugte Ausgestaltungen werden auch in den Unteransprüchen beschrieben.

Ein Ausführungsbeispiel der Erfindung wird nachfolgend an Hand von Figuren erläutert. Es zeigen:
- Figur 1: zwei Teile eines Reservoirmoduls eines Inj ektionsgerätes,
- Figur 2: das Injektionsgerät in einem Längsschnitt,
- Figur 3: eine Kolbenstange nach der Erfindung in einem Längsschnitt,
- Figur 4: einen Teil der Kolbenstange in einer vergrößerten Darstellung, und
- Figur 5: eine Sperreinrichtung für die Kolbenstange in einer Frontansicht.

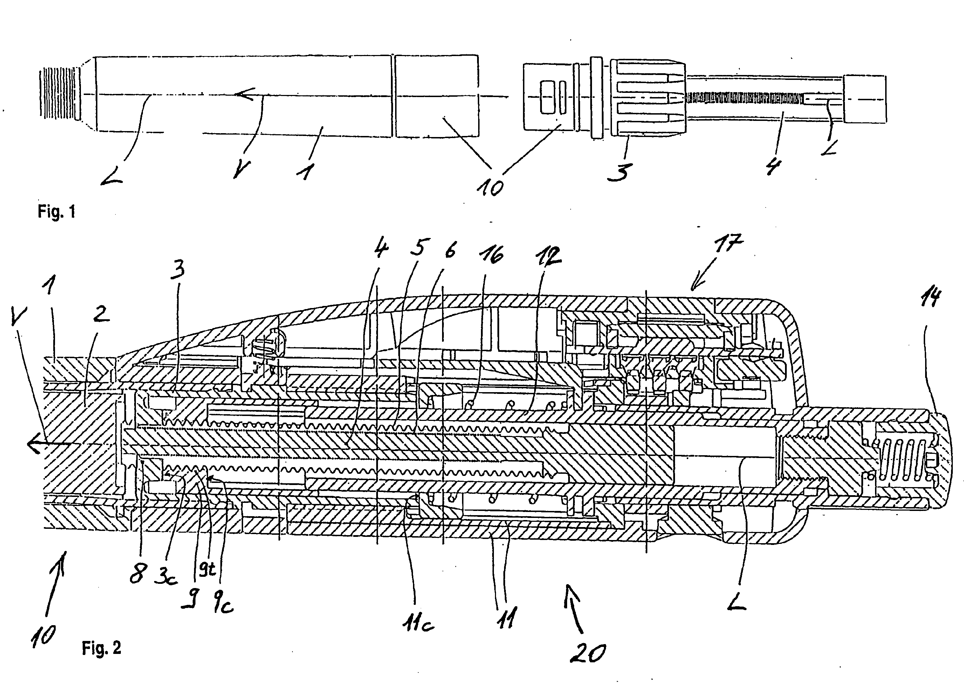

Figur 1 zeigt in einer Ansicht ein Reservoirteil 1 und einen Mechanikhalter 3, die miteinander verbunden werden, um ein Reservoirmodul 10 eines Injektionsgeräts zu bilden. Das Reservoirteil 1 ist im Wesentlichen ein im Querschnitt kreisförmiger Hohlzylinder, der an seinem vorderen Ende einen Verbindungsbereich für eine Verbindung mit einem Nadelhalter für eine Injektionsnadel aufweist. Das Reservoirteil 1 dient der Aufnahme eines Reservoirbehältnisses, das vorzugsweise von einer Ampulle gebildet wird. Das Reservoirbehältnis ist mit einem injizierbaren Produkt, beispielsweise Insulin oder ein Wachstumshormon, gefüllt. Ein Auslass an dem vorderen Ende des Reservoirbehältnisses wird von einer Membran fluiddicht verschlossen. Bei der Befestigung des Nadelhalters an dem vorderen Ende des Reservoirteils 1 durchsticht ein rückwärtiger Teil der Injektionsnadel die Membran, so dass eine Fluidverbindung zwischen der Spitze der Injektionsnadel und dem Reservoir hergestellt ist. Ein dem Auslass axial gegenüberliegendes, hinteres Ende des Reservoirbehältnisses wird fluiddicht von einem Kolben verschlossen, der entlang einer Längsachse L auf den Auslass des Reservoirbehältnisses zu verschiebbar ist, um Produkt aus dem Reservoirbehältnis zu verdrängen. In Figur 1 ist eine Kolbenstange 4 erkennbar, die an einem von dem Reservoirteil 1 abgewandten, hinteren Ende des Mechanikhalters 3 in den Mechanikhalter 3 hineinragt und von dem Mechanikhalter 3 in eine entlang der Längsachse L weisende Vorschubrichtung V auf den Reservoirauslass zu bewegbar gelagert wird.

Figur 2 zeigt einen hinteren Teil des Injektionsgeräts in einem Längsschnitt. Das Injektionsgerät wird von dem Reservoirmodul 10 und einem Dosier- und Betätigungsmodul 20 gebildet. Zu erkennen ist in Figur 1 das hintere Ende des Reservoirmoduls 10. Das Reservoirbehältnis ist vollständig mit Produkt gefüllt, so dass gerade noch der hintere Teil des Kolbens 2 zu sehen ist. Die Kolbenstange 4 bewirkt den Vorschub des Kolbens 2 in die Vorschubrichtung V auf den Reservoirauslass zu, wobei sie mit ihrem vorderen Ende gegen den Kolben 2 drückt. Die Längsachse L ist die Translationsachse des Kolbens 2 und der Kolbenstange 4. Die Kolbenstange 4 wird von dem Mechanikhalter 3 so gehalten, dass sie nach Überwindung eines gewissen Widerstands in die Vorschubrichtung V, nicht jedoch entgegen der Vorschubrichtung V bewegt werden kann.

Die Rückwärtsbewegung der Kolbenstange 4 entgegen der Vorschubrichtung V wird durch das Zusammenwirken einer an der Kolbenstange 4 gebildeten ersten Sperreinrichtung mit einer von dem Mechanikhalter 3 gebildeten zweiten Sperreinrichtung verhindert. Die erste Sperreinrichtung besteht aus zwei Zahnreihen 6, die sich an zwei voneinander abgewandten Seiten der Kolbenstangen 4 axial erstrecken. Die beiden Zahnreihen 6 bestehen aus axial hintereinander in regelmäßiger Teilung angeordneten Sägezähnen. Die zweite Sperreinrichtung besteht aus zwei Sperrzungen 8, die je einer der Zahnreihen 6 gegenüberliegend an dem Mechanikhalter 3 geformt sind und quer zu der Vorschubrichtung V je in eine zwischen zwei benachbarten Zähnen verbleibende Zahnlücke der zugewandten Zahnreihe 6 eingreifen. Die Sägezähne der Zahnreihen 6 sind in Vorschubrichtung V gepfeilt, um die Translationsbewegung der Kolbenstange 4 in die Vorschubrichtung V zuzulassen. Die Sperrzungen 8 werden je von den vorschiebenden Sägezähnen gegen ihre rückstellende Elastizitätskraft nach außen abgebogen. Die rückwärtigen Enden der Sägezähne sind jedoch so geformt, dass durch den Eingriff der Sperrzungen 8 die Rückwärtsbewegung verhindert wird. Im Ausführungsbeispiel weisen die Sägezähne der Zahnreihen 6 an ihren rückwärtigen Enden rechtwinklig zu der Längsachse L. Um die Kolbenstange 4 gegen die in die Zahnlücken eingreifenden Sperrzungen 8 entgegen der Vorschubrichtung V zurückziehen zu können, muss pro Spenzunge 8 eine Widerstandskraft überwunden werden, die deutlich größer als die Elastizitätskraft ist, die einer Bewegung in die Vorschubrichtung V entgegengesetzt wird. Die Sperrzungen 8 müssten in Bezug auf die Kolbenstange 4 zu einem konkaven Bogen verbogen und in solch einer Form ferner auch noch nach außen aus dem Eingriff abgebogen werden.

In dem Mechanikhalter 3 ist ferner ein Dosiseinstellglied 9 aufgenommen. Das Dosiseinstellglied 9 ist als Gewindemutter ausgebildet. Sein Innengewinde 9t steht mit einem Dosiergewinde 5 der Kolbenstange 4 in einem Gewindeeingriff. Die Kolbenstange 4 wird von dem Mechanikhalter 3 in Bezug auf die Längsachse L verdrehgesichert in Vorschubrichtung V geradgeführt. Das Dosiseinstellglied 9 wird von dem Mechanikhalter 3 ebenfalls axial geführt, allerdings kann das Dosiseinstellglied 9 relativ zu dem Mechanikhalter 3 und der Kolbenstange 4 um die Längsachse L eine rotatorische Bewegung ausführen. Die Kolbenstange 4 und das Dosiseinstellglied 9 bilden einen Spindeltrieb, um die zu verabreichende Produktdosis auszuwählen.

Das Reservoirteil 1 und der Mechanikhalter 3 sind verdreh- und verschiebegesichert miteinander verbunden und bilden zusammen das Reservoirmodul 10 des Injektionsgeräts. Das Reservoirmodul 10 umfasst daher auch die mittels der Sperrzungen 8 gehaltene Kolbenstange 4 und das Dosiseinstellglied 9. Das Reservoirteil 1 und der Mechanikhalter 3 bilden zusammen einen vorderen Gehäuseabschnitt des Injektionsgeräts. Mit diesem vorderen Gehäuseabschnitt ist ein hinterer Gehäuseabschnitt 11 verdreh- und verschiebegesichert verbunden. Der hintere Gehäuseabschnitt 11 bildet den Träger eines Dosier- und Betätigungselements 12 und zusammen mit demselben sowie einer Anzeigeeinrichtung 17 und weiteren Teilen des Injektionsgeräts das Dosier- und Betätigungsmodul 20.

Eine Dosier- und Betätigungsvorrichtung des Injektionsgeräts umfasst mit Ausnahme des Dosiseinstellglieds 9 und der Kolbenstange 4 die anderen Komponenten für die Auswahl der Produktdosis und die Betätigung des Injektionsgeräts. Insbesondere umfasst sie das Dosier- und Betätigungselement 12 und die Zähl- und Anzeigeeinrichtung 17 zum Zählen und optischen Anzeigen der ausgewählten Produktdosis. Nicht zuletzt der Zäh1- und Anzeigeeinrichtung 17 wegen ist das Dosier- und Betätigungsmodul 20 ein hochwertiges und daher hochpreisiges Teil des Injektionsgeräts. Während das vergleichsweise preiswerte Reservoirmodul 10 als Einwegmodul konzipiert ist, das nach der Entleerung des Reservoirs einer Entsorgung zugeführt oder bei einem Hersteller wieder aufgearbeitet wird, ist das Dosier- und Betätigungsmodul 20 für die mehrmalige Verwendung mit immer wieder neuen Reservoirmodulen 10 bestimmt.

Für die Auswahl der Produktdosis wird das Dosier- und Betätigungselement 12 um die Längsachse L drehbar und ferner in und gegen die Vorschubrichtung V entlang der Längsachse L geradverschiebbar von dem hinteren Gehäuseabschnitt 11 gelagert. Das Dosier- und Betätigungselement 12 ist hohlzylindrisch und umgibt mit einem vorderen Abschnitt die Kolbenstange 4. Ferner ragt es mit dem vorderen Abschnitt in das hülsenförmige Dosiseinstellglied 9 ein. Ein hinterer Abschnitt des Dosier- und Betätigungselements 12 ragt über ein hinteres Ende des Gehäuseabschnitts 11 hinaus und wird von einer Kappe 14 verschlossen.

Eine Rückstellfeder 16 spannt das Dosier- und Betätigungselement 12 elastisch gegen die Vorschubrichtung V in die in Figur 2 dargestellte hintere Axialposition, die im Folgenden als Ausgangsposition bezeichnet wird. In der Ausgangsposition kann durch Drehung des Dosier- und Betätigungselements 12 um die Längsachse L die Dosisauswahl vorgenommen werden. Anschließend kann ebenfalls aus der Ausgangsposition durch Axialverschiebung des Dosier- und Betätigungselements 12 in die Vorschubrichtung V die Ausschüttung der ausgewählten Produktdosis bewirkt werden.

Das Dosiseinstellglied 9 und das Dosier- und Betätigungselement 12 sind aneinander axial geradgeführt und um die Längsachse L nicht verdrehbar miteinander verbunden. Im Falle einer Drehbewegung des Dosier- und Betätigungselements 12 wird das Dosiseinstellglied wegen der verdrehgesicherten Verbindung mit dem Dosier- und Betätigungselement 12 einerseits und dem Gewindeeingriff mit der relativ zu dem Mechanikhalter 3 nicht verdrehbaren und von der Blockiereinrichtung 8 gehaltenen Kolbenstange 4 andererseits in eine Bewegung versetzt, die sich aus einer rotatorischen Bewegungskomponente um die Längsachse L und einer translatorischen Bewegungskomponente entlang der Längsachse L zusammensetzt.

Das Dosiseinstellglied 9 bildet mit einer rückwärtigen Stirnfläche einen Translationsanschlag 9c für das Dosier- und Betätigungselement 12. Eine Translationsbewegung des Dosier- und Betätigungselements 12 ist relativ zu dem Dosiseinstellglied 9 in die Vorschubrichtung V nur solange möglich, bis das Dosier- und Betätigungselement 12 gegen den Anschlag 9c zu liegen kommt. Sobald an dem Anschlag 9c Kontakt besteht, nimmt das Dosier- und Betätigungselement 12 bei einer weiterführenden Bewegung in die Vorschubrichtung V das Dosiseinstellglied 9 bis in eine vordere Endposition mit, die von einem Translationsanschlag 3c des Mechanikhalters 3 bestimmt wird. Das Dosiseinstellglied 9 wiederum nimmt wegen des Gewindeeingriffs die Kolbenstange 4 mit.
Aus dem in Figur 2 dargestellten Ausgangszustand des Injektionsgerät werden die Dosisauswahl und die Produktausschüttung vorgenommen. Das Dosier- und Betätigungselement 12 nimmt seine Ausgangsposition ein.

Um die zu verabreichende Dosis auszuwählen, wird das Dosier- und Betätigungselement 12 um die Längsachse L verdreht. Wegen der verdrehgesicherten Verbindung nimmt das Dosier- und Betätigungselement 12 bei seiner Drehbewegung das Dosiseinstellglied 9 mit. Diese Dosierdrehbewegung des Dosiseinstellglieds 9 führt wegen des Gewindeeingriffs mit der Kolbenstange 4 zu einer translatorischen Bewegung des Dosiseinstellglieds 9 entlang der Längsachse L entgegen der Vorschubrichtung V. Die translatorische Bewegung führt das Dosiseinstellglied 9 nicht nur relativ zu dem Mechanikhalter 3, sondern auch relativ zu dem Dosier- und Betätigungselement 12 aus. Hierdurch wird ein lichter Abstand zwischen dem von dem Dosiseinstellglied 9 gebildeten Anschlag 9c und einem von dem Dosier- und Betätigungselement 12 gebildeten Gegenanschlag, im Ausführungsbeispiel das freie vordere Ende des Dosier- und Betätigungselements 12, verringert. Im Verlauf der Dosierdrehbewegung entspricht die jeweils eingenommene Axial- und Winkelposition des Dosiseinstellglieds 9 der Produktdosis, die bei einer Betätigung des Dosier- und Betätigungselements 12 ausgeschüttet würde. Die Zähl- und Anzeigeeinrichtung 17 zeigt diese Produktdosis an.

Nach der Auswahl der Produktdosis kann die ausgewählte Produktdosis durch Betätigung des Dosier- und Betätigungselements 12 ausgeschüttet werden. Die Betätigung erfolgt, indem das Dosier- und Betätigungselement 12 in die Vorschubrichtung V gedrückt wird. Dabei legt das Dosier- und Betätigungselement 12 einen ersten Teil seiner Wegstrecke alleine zurück, bis es gegen den Translationsanschlag 9c des Dosiseinstellglieds 9 zu liegen kommt. Im Zuge seiner weiteren Axialbewegung nimmt es dann das Dosiseinstellglied 9 und die Kolbenstange 4 mit, bis das Dosiseinstellglied 9 gegen den von dem Mechanikhalter 3 gebildeten Translationsanschlag 3c stößt. In diesem Moment ist der Ausschütthub beendet. Wird das Dosier- und Betätigungselement 12 losgelassen, so schiebt es aufgrund der gegen die Vorschubrichtung V andrückenden Elastizitätskraft der Rückstellfeder 16 zurück in seine in Figur 2 gezeigte Ausgangsposition, während die Kolbenstange 4 und das Dosiseinstellglied 9 wegen des Eingriffs der Sperrzungen 8 in die Zahnreihen 6 die gerade eingenommene, neue Axialposition beibehalten. Durch die Ausführung des Ausschütthubs oder durch die Rücksetzbewegung der Kolbenstange 4 wird die Zähl- und Anzeigeeinrichtung 17 wieder auf die Minimaldosis zurückgesetzt, im Ausführungsbeispiel genullt. In der wieder erreichten Ausgangsposition des Dosier- und Betätigungselements 12 kann im Rahmen der noch im Reservoir enthaltenen Produktmenge die Produktdosis für die nächste Ausschüttung ausgewählt werden.

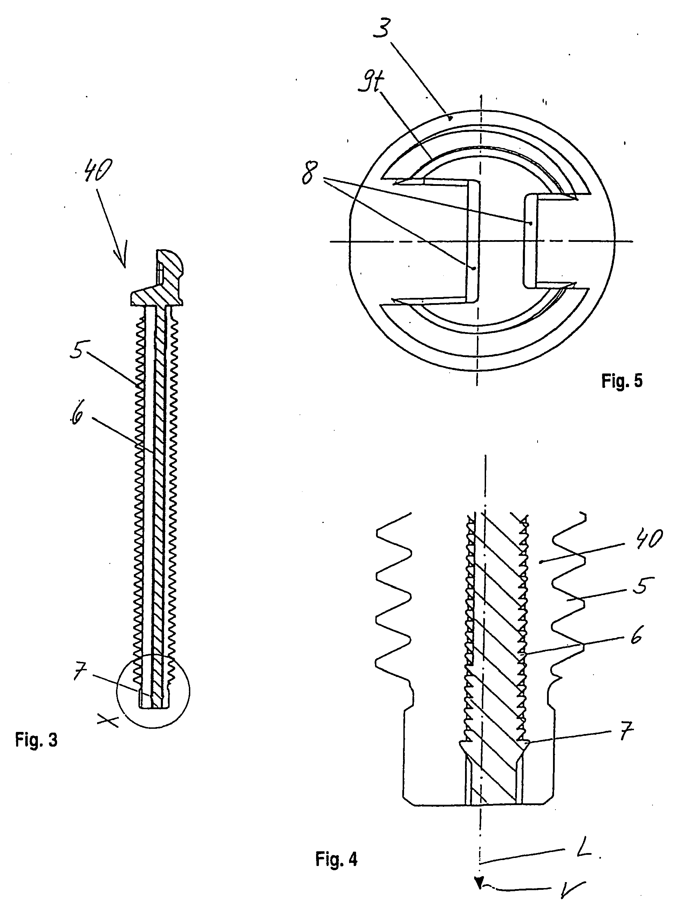

Figur 3 zeigt in einem Längsschnitt eine weiterentwickelte Kolbenstange 40. Soweit die Merkmale in Bezug auf den Vorgang der Dosisauswahl und Produktausschüttung betroffen sind, entspricht die Kolbenstange 40 der in das Injektionsgerät der Figur 2 eingebauten Kolbenstange 4. Insbesondere ist auch die Kolbenstange 40 mit einem Dosiergewinde 5 und einer zwei Zahnreihen 6 aufweisenden ersten Sperreinrichtung versehen. Das Dosiergewinde 5 und die Zahnreihen 6 entsprechen exakt denjenigen bei der Kolbenstange 4 der Figur 2, so dass identische Bezugszeichen verwendet werden. Das besondere Merkmal der Kolbenstange 40 ist die Ausbildung von vergrößerten Sägezähnen 7 je an dem vorderen Ende der beiden Zahnreihen 6. Die beiden vergrößerten Sägezähne 7 bilden je ein Sperrsicherungselement, das in einem Eingriff mit einer der Sperrzungen 8 des Injektionsgeräts der Figur 2 sicherer als die Zahnreihen 6 verhindert, dass die Kolbenstange 40 gänzlich aus dem Mechanikhalter 3 entgegen der Vorschubrichtung V herausgezogen werden kann. Die Zahnreihen 6 und die Sperrsicherungselemente 7 bilden gemeinsam die erste Sperreinrichtung.

Die Sperrsicherungselemente 7 ragen mit ihren rückwärtigen Enden quer zu der Vorschubrichtung V über die Sägezähne der Zahnreihen 6 hinaus. Die rückwärtigen Enden der Sperrsicherungselemente 7 weisen ebenfalls rechtwinklig zur Längsachse L. Auf Grund der in radialer quer zu der Vorschubrichtung längeren Erstreckung der Anschlagflächen an den rückwärtigen Enden der Sperrsicherungselemente 7 im Vergleich zu den Sägezähnen der Zahnreihen 6 wird bei einem Anschlag von einer der Sperrzungen 8 an dem rückwärtige Ende des zugewandten Sperrsicherungselements 7 sicherer eine Rückzugsbewegung der Kolbenstange 40 als bei einem Eingriff der Sperrzungen 8 in die Lücken der regulären Sägezähne der Zahnreihen 6 verhindert.

Andererseits lassen es auch die Sperrsicherungselemente 7 zu, dass die Kolbenstangen 40 von hinten in den Mechanikhalter 3 eingesetzt werden kann. Bei dem Einsetzen wird die Kolbenstange 40 in die Vorschubrichtung V durch eine zwischen den Sperrzungen 8 in dem Mechanikhalter 3 verbleibende Öffnung geschoben. Bei der Bewegung der Kolbenstange 40 in die Vorschubrichtung V gleiten die Sperrsicherungselemente 7 an den Sperrzungen 8 entlang. Bei dem Entlanggleiten werden die Sperrzungen 8 auf Grund der sich allmählich verbreiternden Sperrsicherungselemente 7 elastisch in die Vorschubrichtung V und nach radial auswärts abgebogen bis die Sperrsicherungselemente 7 mit ihren rückwärtigen Enden über die Sperrzungen 8 geglitten sind. Auf Grund ihrer Elastizität schnappen die Sperrzungen 8 in diesem Moment wieder nach radial einwärts in Zahnlücken vor, die zwischen den rückwärtigen Enden der Sperrsicherungselemente 7 und dem je nächst benachbarten, ersten Sägezahn der Zahnreihen 6 verbleiben. Die beiden betreffenden Zahnlücken sind in radialer Richtung genauso tief wie die regulären Zahnlücken der Zahnreihen 6. Da jedoch die rückwärtigen, die Anschlagflächen bildenden Enden der Sperrsicherungselemente 7 quer zu der Vorschubrichtung V über die von den Sägezähnen der Zahnreihen 6 gebildeten rückwärtigen Anschlagsflächen vorstehen, ist die auf Formschluss beruhende Sperrwirkung der Sperrsicherungselemente 7 besonders sicher. Eine Rückzugsbewegung der Kolbenstange 40 entgegen der Vorschubsrichtung V würde die Zerstörung der zweiten Sperreinrichtung 8 oder, was praktisch ausgeschlossen werden kann, die Zerstörung der Sperrsicherungselemente 7 erfordern. In Bezug auf den Eingriff der zweiten Sperreinrichtung 8 in beiden Zahnreihen kann mit weit geringerer Sicherheit gewährleistet werden, dass die für eine Rückzugsbewegung der Kolbenstange 40 zu überwindende Widerstandskraft unweigerlich die Zerstörung der zweiten Sperreinrichtung 8 oder/und der Zahnreihen 6 zur Folge haben muss. In Bezug auf diesen Eingriff besteht durchaus die Gefahr, dass die Sperrzungen 8 nicht nur bei einer Bewegung der Kolbenstange 40 in die Vorschubrichtung V, sondern auch bei einer Bewegung entgegen der Vorschubrichtung V elastisch in sich verbogen und anschließend nach außen abgebogen werden, bis sie an den rückwärtigen Enden der Sägezähne der beiden Zahnreihen 6 abrutschen. Die Sperrzungen 8 können auf Grund ihrer grundsätzlichen Biegenachgiebigkeit möglicherweise so stark verbogen werden, dass solch ein Abrutschen ermöglicht wird. Bei einem Eingriff unmittelbar hinter die beiden Sperrsicherungselemente 7 müssten die Sperrzungen 8 vor solch einem Abrutschen, sollten sie nicht zerstört werden, jedoch deutlich stärker verbogen werden. Eine zur Überwindung der Sperrsicherungselemente 7 zu überwindende Sperrkraft ist daher deutlich größer als die Widerstandskraft, die zur Überwindung der Sägezähne der Zahnreihen 6 aufgebracht werden muss. Angestrebt wird, dass die Kolbenstange 4 nur durch Zerstörung der zweiten Sperreinrichtung 8 oder/und der Sperrsicherungselemente 7 von dem Reservoirmodul 10 getrennt werden kann.

Figur 5 zeigt die zweite Sperreinrichtung 8 in einer Ansicht auf ihre Vorderseite. Zu erkennen sind insbesondere die beiden Sperrzungen 8, die quer zu der Vorschubrichtung nach einwärts von dem Hülsenkörper des Mechanikhalters 3 vorragen. Zu erkennen ist ferner auch das Gewinde 9t des Dosiseinstellglieds 9.

Der Mechanikhalter 3 bildet die zweite Sperreinrichtung 8 in einem Stück. Im Ausführungsbeispiel ist der Mechanikhalter 3 ein Spritzgussteil. Auch die Kolbenstange 40 ist ein Spritzgussteil und bildet ihr Dosiergewinde 5, die beiden Zahnreihen 6 und die Sperrsicherungselemente 7 in einem Stück.

In Bezug auf die Zahnreihen 6 und die Sperrsicherungselemente 7 ist noch darauf hinzuweisen, dass diese funktionalen Teile der Kolbenstange 40 in axialen Nuten angeordnet sind, die sich an einander gegenüberliegenden Seiten der Kolbenstange 40 parallel zu der Längsachse L erstrecken und das Dosiergewinde 5 durchbrechen. Die Zahnreihen 6 und die Sperrsicherungselemente 7 stehen hinter dem Gewindegrund des Dosiergewindes 5 zurück, so dass sie das Verschrauben der Kolbenstange 40 mit dem Dosiseinstellglied 9 und im Betrieb die Dosierdrehbewegung des Dosiseinstellglieds 9 nicht behindern.

## Patentansprüche

1. Verabreichungsgerät zur Verabreichung eines fluiden Produkts, das Verabreichungsgerät umfassend:
a) ein Gehäuse (1, 3, 11) mit einem Reservoir für das Produkt,
b) einen Kolben (2), der in dem Reservoir in eine Vorschubrichtung (V) verschiebbar aufgenommen ist, um Produkt auszuschütten,
c) eine in die Vorschubrichtung (V) auf den Kolben wirkende Kolbenstange (40), die eine erste Sperreinrichtung (6, 7) bildet,
d) eine relativ zu dem Gehäuse (1, 3, 11) gegen die Vorschubrichtung (V) nicht bewegbare zweite Sperreinrichtung (8),
e) eine in Vorschubrichtung (V) erstreckte, von hintereinander angeordneten ersten Sperrelementen gebildete Sperrelementreihe (6), die von einer der Sperreinrichtungen (6, 7, 8) gebildet wird,
f) und ein zweites Sperrelement (8), das von der anderen der Sperreinrichtungen (6, 7, 8) gebildet wird und mit der Sperrelementreihe (6) in einem Eingriff ist, in dem es in eine Sperrelementlücke der Sperrelementreihe (6) eingreift,
g) wobei das zweite Sperrelement (8) durch eine in die Vorschubrichtung (V) gerichtete Bewegung der Kolbenstange (40) gegen eine Elastizitätskraft aus dem Eingriff bewegt wird, aber einer gegen die Vorschubrichtung (V) gerichteten Bewegung der Kolbenstange (40) in dem Eingriff eine Widerstandskraft entgegensetzt, die größer als die Elastizitätskraft ist, um eine gegen die Vorschubrichtung (V) gerichtete Bewegung der Kolbenstange (40) zu verhindern,
h) und wobei die erste Sperreinrichtung (6) an einem in Vorschubrichtung (V) vorderen Ende oder/und die zweite Sperreinrichtung (8) an einem in Vorschubrichtung (V) hinteren Ende ein Sperrsicherungselement (7) bildet, das einer gegen die Vorschubrichtung (V) gerichteten Bewegung der Kolbenstange (40) eine Sperrkraft entgegensetzt, die größer als die Widerstandskraft ist.

2. Verabreichungsgerät nach Anspruch 1, **dadurch gekennzeichnet, dass** das Sperrsicherungselement (7) an einem Ende der Sperrelementreihe (6) gebildet ist.

3. Verabreichungsgerät nach dem vorhergehenden Anspruch, **dadurch gekennzeichnet, dass** das Sperrsicherungselement (7) quer zu der Vorschubrichtung (V) über die Sperrelementreihe (6) vorragt oder biegesteifer als die ersten Sperrelemente ist.

4. Verabreichungsgerät nach einem der vorhergehenden Ansprüche, **dadurch gekennzeichnet, dass** die Sperrelementreihe (6) eine Zahnreihe ist.

5. Verabreichungsgerät nach einem der vorhergehenden Ansprüche, **dadurch gekennzeichnet, dass** das Sperrsicherungselement (7) die Sperrkraft entweder in einem Eingriff mit dem zweiten Sperrelement (8) oder in einem Eingriff mit der Sperrelementreihe (6) erzeugt.

6. Verabreichungsgerät nach dem vorhergehenden Anspruch, **dadurch gekennzeichnet, dass** die Zahnreihe (6) von Sägezähnen gebildet wird und das Sperrsicherungselement (7) eine Anschlagfläche für das zweite Sperrelement (8) unmittelbar an einem verjüngten Ende von einem der Sägezähne der Zahnreihe (6) bildet, wobei die Anschlagfläche gegen die Vorschubrichtung (V) weist, wenn die Kolbenstange (40) das Sperrsicherungselement (7) bildet, und wobei die Anschlagfläche in die Vorschubrichtung (V) weist, wenn die zweite Sperreinrichtung das Sperrsicherungselement bildet.

7. Verabreichungsgerät nach einem der vorhergehenden Ansprüche, **dadurch gekennzeichnet, dass** die Sperrelemente der Sperrelementreihe (6) je eine Anschlagfläche für das zweite Sperrelement (8) bilden, um durch Formschluss eine gegen die Vorschubrichtung (V) gerichtete Bewegung der Kolbenstange (40) zu verhindern, und auch das Sperrsicherungselement (7) eine Anschlagfläche bildet, um durch Formschluss eine gegen die Vorschubrichtung (V) gerichtete Bewegung der Kolbenstange (4) zu verhindem, und dass die Anschlagfläche des Sperrsicherungselements (7) von der Anschlagsfläche des nächst benachbarten Sperrelements der Sperrelementreihe (6) zumindest im Wesentlichen, vorzugsweise genau, den gleichen in Vorschubrichtung gemessenen Abstand hat wie je zwei Anschlagflächen von einander nächst benachbarten Sperrelementen der Sperrelementreihe (6).

8. Verabreichungsgerät nach einem der vorhergehenden Ansprüche, **dadurch gekennzeichnet, dass** die erste Sperreinrichtung (6) die Sperrelementreihe (6) bildet.

9. Verabreichungsgerät nach Anspruch 1, **dadurch gekennzeichnet, dass** diejenige der Sperreinrichtungen, die das zweite Sperrelement bildet, auch das Sperrsicherungselement bildet und das Sperrsicherungselement quer zu der Vorschubrichtung (V) über das zweite Sperrelement vorragt oder biegesteifer als das zweite Sperrelement ausgebildet ist.

10. Verabreichungsgerät nach einem der vorhergehenden Ansprüche, **dadurch gekennzeichnet, dass** das Sperrsicherungselement (7) ein Sägezahn ist, der sich in oder gegen die Vorschubrichtung (V) pfeilförmig bis zu einem Sägezahnende verbreitert und an dem Sägezahnende eine in oder gegen die Vorschubrichtung (V) weisende Anschlagfläche für das zweite Sperrelement (8) bildet.

11. Verabreichungsgerät nach einem der vorhergehenden Ansprüche, **dadurch gekennzeichnet, dass** das Sperrsicherungselement (7) die Sperrkraft in einem Eingriff mit einem Sicherungsgegenelement (8) erzeugt und eine in die Vorschubrichtung (V) gerichtete Bewegung über das Sicherungsgegenelement (8) hinweg zulässt, damit das zweite Sperrelement (8) in den Eingriff mit der Sperrelementreihe (6) gebracht werden kann.

12. Verabreichungsgerät nach einem der vorhergehenden Ansprüche, **dadurch gekennzeichnet, dass** die Kolbenstange (40) einen Gewindeabschnitt (5) und in dem Gewindeabschnitt (5) eine in Vorschubrichtung (V) erstreckte Ausnehmung aufweist, in der die erste Sperreinrichtung (6) gebildet ist.

13. Verabreichungsgerät nach dem vorhergehenden Anspruch, **dadurch gekennzeichnet, dass** das Sperrsicherungselement (7) in der Ausnehmung ausgebildet ist und quer zu der Vorschubrichtung (V) nicht über einen Gewindegrund des Gewindebschnittes (5) vorsteht.

14. Verabreichungsgerät nach einem der vorhergehenden Ansprüche, **dadurch gekennzeichnet, dass** das Verabreichungsgerät ein Reservoirmodul (10) und ein lösbar mit dem Reservoirmodul (10) verbundenes Dosier- und Betätigungsmodul (30) umfasst, wobei das Reservoirmodul (10) einen vorderen Gehäuseabschnitt (1, 3) des Gehäuses (1, 3, 11) bildet, der das Reservoir, die zweite Sperreinrichtung (8) und die in dem Eingriff mit der zweiten Sperreinrichtung (8) befindliche Kolbenstange (40) umfasst, und wobei das Dosier- und Betätigungsmodul (30) eine hinteren Gehäuseabschnitt (11) des Gehäuses (1, 3, 11) mit einer Dosier- und Antriebseinrichtung (12) des Verabreichungsgerät umfasst.

15. Reservoirmodul für das Verabreichungsgerät nach dem vorhergehenden Anspruch, das Reservoirmodul (10) umfassend:
a) den vorderen Gehäuseabschnitt (1, 3) des Gehäuses (1, 3, 11), der das Reservoir mit dem darin aufgenommenen Kolben (2), die zweite Sperreinrichtung (8) und eine Verbindungseinrichtung zur Herstellung der Verbindung mit dem Dosier- und Betätigungsmodul (30) aufweist,
b) das Dosiseinstellglied (9), das von dem vorderen Gehäuseabschnitt (1, 3) bewegbar aufgenommen wird, um eine Dosierbewegung und eine die Ausschüttung des Produkts bewirkende Ausschüttbewegung auszuführen,
c) und die Kolbenstange (40), die mit dem Dosiseinstellglied (9) verbunden und mit der zweiten Sperreinrichtung (8) in dem Eingriff ist.

## Claims

1. An administration device for the administration of a fluid product, the administration device comprising:
a) a housing (1, 3, 11) with a reservoir for the product,
b) a piston (2) which is accommodated in the reservoir displaceably in an advance direction to expel product,
c) a piston rod (40) which acts on the piston in the advance direction (V) and which forms a first locking device (6, 7),
d) a second locking device (8) which is not movable relative to the housing (1, 3, 11) in opposite relationship to the advance direction (V),
e) a locking element row (6) which is extended in the advance direction (V) and which is formed by successively arranged first locking elements and which is formed by one of the locking devices (6, 7, 8),
f) and a second locking element (8) which is formed by the other of the locking devices (6, 7, 8) and is in engagement with the locking element row (6) by engaging into a locking element gap in the locking element row (6),
g) wherein the second locking element (8) is moved out of its engagement against an elasticity force by a movement of the piston rod (40) directed in the advance direction (V) but opposes a resistance force which is greater than the elasticity force to a movement of the piston rod (40) directed in opposite relationship to the advance direction (V) in the engagement condition in order to prevent a movement of the piston rod (40) directed in opposite relationship to the advance direction (V),
h) and wherein the first locking device (6) at a front end in the advance direction (V) and/or the second locking device (8) at a rear end in the advance direction (V) forms a locking securing element (7) which opposes a locking force which is greater than the resistance force to a movement of the piston rod (40) directed in opposite relationship to the advance direction (V).

2. An administration device according to claim 1 **characterised in that** the locking securing element (7) is formed at an end of the locking element row (6).

3. An administration device according to the preceding claim **characterised in that** the locking securing element (7) projects transversely with respect to the advance direction (V) beyond the locking element row (6) or is flexurally stiffer than the first locking elements.

4. An administration device according to one of the preceding claims **characterised in that** the locking element row (6) is a row of teeth.

5. An administration device according to one of the preceding claims **characterised in that** the locking securing element (7) produces the locking force either in a condition of engagement with the second locking element (8) or in a condition of engagement with the locking element row (6).

6. An administration device according to the preceding claim **characterised in that** the row of teeth (6) is formed by sawteeth and the locking securing element (7) forms an abutment surface for the second locking element (8) directly at a tapered end of one of the sawteeth of the row of teeth (6), wherein the abutment surface faces in opposite relationship to the advance direction (V) when the piston rod (40) forms the locking securing element (7) and wherein the abutment surface faces in the advance direction (V) when the second locking device forms the locking securing element.

7. An administration device according to one of the preceding claims **characterised in that** the locking elements of the locking element row (6) each form a respective abutment surface for the second locking element (8) to prevent by positively locking engagement a movement of the piston rod (40) directed in opposite relationship to the advance direction (V), and the locking securing element (7) also forms an abutment surface to prevent by positively locking engagement a movement of the piston rod (4) directed in opposite relationship to the advance direction (V), and that the abutment surface of the locking securing element (7) is at least substantially and preferably precisely at the same spacing measured in the advance direction from the abutment surface of the next adjacent locking element of the locking element row (6), as two respective abutment surfaces of mutually closest adjacent locking elements of the locking element row (6).

8. An administration device according to one of the preceding claims **characterised in that** the first locking device (6) forms the locking element row (6).

9. An administration device according to claim 1 **characterised in that that** one of the locking devices that forms the second locking element also forms the locking securing element and the locking securing element projects transversely with respect to the advance direction (V) beyond the second locking element or is flexurally stiffer than the second locking element.

10. An administration device according to one of the preceding claims **characterised in that** the locking securing element (7) is a sawtooth which widens in an arrow shape to a sawtooth end in or in opposite relationship to the advance direction (V) and at the sawtooth end forms an abutment surface for the second locking element (8), said abutment surface facing in or in opposite relationship to the advance direction (V).

11. An administration device according to one of the preceding claims **characterised in that** the locking securing element (7) produces the locking force in a condition of engagement with a securing counterpart element (8) and permits a movement, directed in the advance direction (V), beyond the securing counterpart element (8) so that the second locking element (8) can be brought into engagement with the locking element row (6).

12. An administration device according to one of the preceding claims **characterised in that** the piston rod (40) has a screwthreaded portion (5) and in the screwthreaded portion (5) a recess which is extended in the advance direction (V) and in which the first locking device (6) is formed.

13. An administration device according to the preceding claim **characterised in that** the locking securing element (7) is formed in the recess and does not project transversely with respect to the advance direction (V) beyond the bottom of a screwthread of the screwthreaded portion (5).

14. An administration device according to one of the preceding claims **characterised in that** the administration device includes a reservoir module (10) and a dosing and actuating module (30) releasably connected to the reservoir module (10), wherein the reservoir module (10) forms a front housing portion (1, 3) of the housing (1, 3, 11), which includes the reservoir, the second locking device (8) and the piston rod (40) which is in engagement with the second locking device (8), and wherein the dosing and actuating module (30) includes a rear housing portion (11) of the housing (1, 3, 11) with a dosing and drive device (12) of the administration device.

15. A reservoir module for the administration device according to the preceding claim, the reservoir module (10) comprising:
a) the front housing portion (1, 3) of the housing (1, 3, 11), which has the reservoir with the piston (2) accommodated therein, the second locking device (8) and a connecting device for making the connection to the dosing and actuating module (30),
b) the dose setting member (9) which is movably accommodated by the front housing portion (1, 3) to perform a dosing movement and an expulsion movement to expel the product,
c) and the piston rod (40) which is connected to the dose setting member (9) and is in engagement with the second locking device (8).

## Revendications

1. Appareil d'administration destiné à administrer un produit fluide, l'appareil d'administration comprenant :
a) un boîtier (1, 3, 11) avec un réservoir pour le produit,
b) un piston (2), qui est logé de manière coulissante dans le réservoir dans une direction d'avance (V), afin de verser le produit,
c) une tige de piston (40) agissant sur le piston dans la direction d'avance (V), formant un premier dispositif de blocage (6, 7),
d) un second dispositif de blocage (8) non mobile par rapport au boîtier (1, 3, 11) à l'opposé de la direction d'avance (V),
e) une série d'éléments de blocage (6) s'étendant dans la direction d'avance (V), formée par des premiers éléments de blocage agencés les uns derrière les autres, qui est constituée de l'un des dispositifs de blocage (6, 7, 8),
f) et un second élément de blocage (8) qui est formé par l'autre des dispositifs de blocage (6, 7, 8) et est en prise avec la série d'éléments de blocage (6), en ce qu'il s'engage dans un intervalle entre les éléments de blocage de la série d'éléments de blocage (6),
g) le second élément de blocage (8) étant déplacé hors de prise par un déplacement orienté dans la direction d'avance (V) de la tige de piston (40) à l'encontre de la force d'élasticité, et opposant une force de résistance, plus importante que la force d'élasticité, à un déplacement de la tige de piston (40) en prise orienté à l'encontre de la direction d'avance (V), afin d'empêcher un déplacement de la tige de piston (40) orienté à l'encontre de la direction d'avance (V),
h) et le premier dispositif de blocage (6) formant sur une extrémité avant dans la direction d'avance (V) et/ou le second dispositif de blocage (8) formant sur une extrémité arrière dans la direction d'avance (V) un élément de fixation de blocage (7) qui oppose une force de blocage à un déplacement orienté à l'encontre de la direction d'avance (V) de la tige de piston (40) plus importante que la force de résistance.

2. Appareil d'administration selon la revendication 1, **caractérisé en ce que** l'élément de fixation de blocage (7) est formé à une extrémité de la série d'éléments de blocage (6).

3. Appareil d'administration selon la revendication précédente, **caractérisé en ce que** l'élément de fixation de blocage (7) fait saillie au-dessus de la série d'éléments de blocage (6) de manière transversale à la direction d'avance (V) ou est plus résistant à la flexion que les premiers éléments de blocage.

4. Appareil d'administration selon l'une quelconque des revendications précédentes, **caractérisé en ce que** la série d'éléments de blocage (6) est une série de dents.

5. Appareil d'administration selon l'une quelconque des revendications précédentes, **caractérisé en ce que** l'élément de fixation de blocage (7) produit la force de blocage soit en prise avec le second élément de blocage (8) soit en prise avec la série d'éléments de blocage (6).

6. Appareil d'administration selon la revendication précédente, **caractérisé en ce que** la série de dents (6) est formée de dents de scie et l'élément de fixation de blocage (7) forme une surface de butée pour le second élément de blocage (8) directement sur une extrémité réduite de l'une des dents de scie de la série de dents (6), la surface de butée étant orientée à l'opposé de la direction d'avance (V), lorsque la tige de piston (40) forme l'élément de fixation de blocage (7), et la surface de butée étant orientée dans la direction d'avance (V), lorsque le second dispositif de blocage forme l'élément de fixation de blocage.

7. Appareil d'administration selon l'une quelconque des revendications précédentes, **caractérisé en ce que** les éléments de blocage de la série d'éléments de blocage (6) forment respectivement une surface de butée pour le second élément de blocage (8), afin d'empêcher un déplacement de la tige de piston (40) orienté à l'encontre de la direction d'avance (V) par complémentarité de forme, et l'élément de fixation de blocage (7) forme une surface de butée, afin d'empêcher un déplacement de la tige de piston (4) orienté à l'encontre de la direction d'avance (V) par complémentarité de forme, et **en ce que** la surface de butée de l'élément de fixation de blocage (7) présente au moins essentiellement, de préférence exactement, le même écart mesuré dans la direction d'avance depuis la surface de butée de l'élément de blocage voisin de la série d'éléments de blocage (6) que deux surfaces de butée d'éléments de blocage voisins l'un de l'autre de la série d'éléments de blocage.

8. Appareil d'administration selon l'une quelconque des revendications précédentes, **caractérisé en ce que** le premier dispositif de blocage (6) forme la série d'éléments de blocage (6).

9. Appareil d'administration selon la revendication 1, **caractérisé en ce que** le dispositif de blocage qui forme le second élément de blocage, forme également l'élément de fixation de blocage et l'élément de fixation de blocage fait saillie au-dessus du second élément de blocage de manière transversale par rapport à la direction d'avance (V) ou est conçu plus résistant à la flexion que le second élément de blocage.

10. Appareil d'administration selon l'une quelconque des revendications précédentes, **caractérisé en ce que** l'élément de fixation de blocage (7) est une dent de scie, qui s'élargit dans ou à l'encontre de la direction d'avance (V) en forme de flèche jusqu'à une extrémité de dent de scie et forme à l'extrémité de dent de scie une surface de butée orientée dans ou à l'encontre de la direction d'avance (V) pour le second élément de blocage (8).

11. Appareil d'administration selon l'une quelconque des revendications précédentes, **caractérisé en ce que** l'élément de fixation de blocage (7) produit la force de blocage en prise avec un contre-élément de fixation (8) et autorise un déplacement orienté dans la direction d'avance (V) au-delà du contre-élément de fixation (8), afin que le second élément de blocage (8) soit mis en prise avec la série d'éléments de blocage (6).

12. Appareil d'administration selon l'une quelconque des revendications précédentes, **caractérisé en ce que** la tige de piston (40) présente une section filetée (5) et, dans la section filetée (5), un évidement s'étendant dans la direction d'avance (V), dans lequel le premier dispositif de blocage (6) est formé.

13. Appareil d'administration selon l'une quelconque des revendications précédentes, **caractérisé en ce que** l'élément de fixation de blocage (7) est réalisé dans l'évidement et ne fait pas saillie au-dessus d'un fond de filet de la section filetée (5), de manière transversale à la direction d'avance (V).

14. Appareil d'administration selon l'une quelconque des revendications précédentes, **caractérisé en ce que** l'appareil d'administration comprend un module de réservoir (10) et un module de dosage et d'actionnement (30) relié de manière amovible au module de réservoir (10), le module de réservoir (10) formant une section de boîtier avant (1, 3) du boîtier (1, 3, 11), qui comprend le réservoir, le second dispositif de blocage (8) et la tige de piston (40) se trouvant en prise avec le second dispositif de blocage (8), et le module de dosage et d'actionnement (30) comprenant une section de boîtier arrière (11) du boîtier (1, 3, 11) avec un dispositif de dosage et d'entraînement (12) de l'appareil d'administration.

15. Module de réservoir pour l'appareil d'administration selon la revendication précédente, le module de réservoir (10) comprenant :
a) le segment de boîtier avant (1, 3) du boîtier (1, 3, 11), qui présente le réservoir avec le piston (2) logé à l'intérieur, le second dispositif de blocage (8) et un dispositif de raccordement destiné à réaliser le raccordement avec le module de dosage et d'actionnement (30),
b) l'organe de réglage de dose (9) qui est logé de manière mobile par la section de boîtier avant (1, 3), afin de réaliser un déplacement pour le dosage et un déplacement pour le déversement permettant de verser le produit,
c) et la tige de piston (40) qui est reliée à l'organe de réglage de dose (9) et est en prise avec le second dispositif de blocage (8).
